# EUROPEAN PATENT APPLICATION

(11) **EP 2 156 804 A1**
(43) Date of publication of application: **24.02.2010**
(21) Application number: 09010573.5
(22) Date of filing: 17.08.2009
(51) Int. Cl.: A61B 18/24, A61B 5/00, A61B 17/00, A61B 18/20

(54) **Laser treatment apparatus**

(30) Priority: 18.08.2008 JP 2008209526
(71) Applicant: FUJIFILM Corporation, Minato-ku Tokyo (JP)
(72) Inventor: Nariyuki, Fumito, Ashigarakami-gun, Kanagawa (JP); Nabeta, Toshiyuki, Ashigarakami-gun, Kanagawa (JP); Mogi, Shusuke, Minato-ku, Tokyo (JP)
(74) Representative: Höhfeld, Jochen

(57) **Abstract**

A laser treatment apparatus (11) is provided with a treatment probe (24), an observation probe (23), an image processing section (67), a lesion disappearance degree judgment section (69), and a CPU (68). The treatment probe and the observation probe are disposed in a catheter (21) inserted into a blood vessel (12). The observation probe scans an inner wall of the blood vessel to obtain a tomographic image signal therefrom. The image processing section produces a tomographic image (72) from the tomographic image signal. The lesion disappearance degree judgment section analyzes the tomographic image and judges whether or not a lesion (17) exists. The CPU controls radiation of a laser beam from the treatment probe based on a judgment result of the lesion disappearance degree judgment section.

## Description

### BACKGROUND OF THE INVENTION

### 1. Field of the Invention

The present invention relates to a laser treatment apparatus that irradiates a lesion on an arterial wall with a laser beam, and more particularly relates to the laser treatment apparatus by which a doctor provides therapy while monitoring tomographic images of the lesion.

### 2. Description Related to the Prior Art

It is known that stenosis or embolization of a coronary artery brings about angina pectoris or myocardial infarction. This is atherosclerosis in which the stenosis and the embolization of the coronary artery are mainly caused by plaque, which is predominantly composed of lipid e.g. cholesterol, adhering to an inner lining of an arterial wall and building up thereon.

As a treatment for the angina pectoris and the myocardial infarction, is known percutaneous catheter intervention (PCI). In the PCI, a catheter is introduced into the coronary artery to ablate the plaque or dilate a blood vessel of a narrowed area or occlusion area (hereinafter called treatment area). For example, a bare metal stent or a drug-eluting stent dwells in the narrowed area, or a balloon catheter expands the blood vessel. In the case of the embolization or in-stent restenosis, laser angioplasty is adopted.

In the laser angioplasty, the plaque is removed by irradiation with a laser beam. A thermal laser such as an argon laser or a CO₂ laser has been used, but thermal laser irradiation can unintentionally damage normal tissue surrounding the lesion. Accordingly, a laser treatment apparatus using a nonthermal laser such as an ultraviolet excimer laser is proposed in recent years (refer to U.S. Patent No. 5,093,877 and European Patent Application No. 0355200).

U. S. Patent No. 7, 238, 178 (corresponding to Japanese Patent Laid-Open Publication No. 2005-230549) and WO 98/38907 (corresponding to Japanese Patent Application Publication No. 2001-515382) disclose to combine a therapeutic laser and a probe unit for OCT (optical coherence tomography) at a distal end of a catheter assembly, which is introduced into the coronary artery. A combination of the therapeutic laser and the probe unit allows a doctor to perform the laser angioplasty while monitoring a treatment area. This combination also has the advantages of offering clear tomographic images by the OCT and preventing X-ray irradiation.

In the laser angioplasty, the doctor has to provide therapy with extreme caution and discretion while frequently checking the tomographic images whenever the laser beam is applied for a short while, because the treatment area is very small and the arterial wall is thin. In short, the laser angioplasty requires the doctor to have high and fine technique. Accordingly, it is desired to take measures for improving safety in the therapy and reducing a technical burden of the doctor.

The above patent documents describe only combining the therapeutic laser and the OCT, and do not disclose concrete structure for connecting the both.

### SUMMARY OF THE INVENTION

An object of the present invention is to provide a laser treatment apparatus that improves safety and reduces a technical burden of a doctor.

To achieve the foregoing objects, a laser treatment apparatus according to the present invention is provided with a treatment probe, an observation probe, an image processing section, a lesion disappearance degree judgment section, and a probe controller. The treatment probe is disposed in a catheter assembly. The treatment probe applies a laser beam to a lesion in a treatment area in a body cavity for reaction to material of the lesion. The observation probe is disposed in the catheter assembly together with the treatment probe. The observation probe applies electromagnetic radiation to the treatment area and receives reflected electromagnetic radiation from the treatment area. The image processing section produces a tomographic image of the treatment area based on the reflected electromagnetic radiation from the observation probe. The lesion disappearance degree judgment section analyzes the tomographic image and judges the degree of disappearance of the lesion by the reaction. The probe controller controls radiation of the laser beam in response to a judgment result of the lesion disappearance degree judgment section.

The laser treatment apparatus may further include a light source for emitting low-coherent light as the electromagnetic radiation, a light splitter, a frequency modulator, a scanner, and an interference light generator. The light splitter splits the low-coherent light into reference light and signal light. The signal light is incident on the observation probe. The frequency modulator slightly varies the frequency of the reference light. The scanner scans the treatment area with the signal light by rotating the observation probe. Reflected signal light from the treatment area is incident on the observation probe. The interference light generator generates interference light by interference of the reflected signal light from the observation probe and the reference light. The image processing section produces the tomographic image from the interference light. The laser treatment apparatus may further include an optical delay section for varying an optical path length of the reference light to vary imaging depth thereof. It is preferable that each of the treatment probe and the observation probe have an optical fiber and a reflective optical member attached at a tip of the optical fiber. It is preferable that the catheter assembly include a transparent catheter. It is preferable that the treatment probe and the observation probe be movable in the catheter.

The laser treatment apparatus may further include a monitor for displaying the tomographic image.

In the laser treatment apparatus, the probe controller may control a laser radiation condition that includes at least one of switching radiation of the laser beam, a laser beam irradiation position, an amount of irradiation of the laser beam per unit of time, and a choice of wavelengths of the laser beam.

In the laser treatment apparatus, the disappearance of the lesion is judged based on luminance of the treatment area by analyzing the tomographic image. More particularly, to judge the disappearance of the lesion, a reference value of the luminance of the lesion is obtained, and luminance of the treatment area is compared with the reference value.

The laser treatment apparatus may further include a designation section for designating the treatment area in the tomographic image.

The probe controller has an automatic mode for automatically controlling the treatment probe. In the automatic mode, the treatment probe continues applying the laser beam while the laser exists, and stops applying the laser beam when the lesion disappearance degree judgment section judges that the lesion has disappeared.

The probe controller has an assistance mode for assisting laser radiation operation of the treatment probe. In the assistance mode, the treatment probe permits the laser radiation operation while the lesion exists, and prohibits the laser radiation operation when the lesion disappearance degree judgment section judges that the lesion has disappeared. The laser treatment apparatus may further include a warning section that externally notifies the judgment result of the lesion disappearance degree judgment section.

The laser beam may have a wavelength of 5.75 µm.

The laser beam may have a wavelength between or equal to 1100 µm and 1800 µm.

The laser beam may radiate from a semiconductor laser or a free electron laser.

The treatment probe may radiate the laser beam with a wavelength of at least one of 193 nm, 248 nm, 308 nm, and 351 nm.

The treatment probe may radiate one of a plurality of laser beams with different wavelengths.

According to the present invention, it is possible to improve safety in laser angioplasty and reduce a technical burden of the doctor.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is a perspective view schematically showing a catheter assembly of a laser treatment apparatus;
Fig. 2 is a sectional view of the catheter assembly;
Fig. 3 is a block diagram of the laser treatment apparatus;
Fig. 4 is a diagram showing observation images displayed on a monitor screen;
Fig. 5 is a flowchart of laser angioplasty in an assistance mode;
Fig. 6 is an explanatory view showing an example of the observation image at steps S1 and S2 of Fig. 5;
Fig. 7 is an explanatory view showing an example of the observation image at a step S3 of Fig. 5;
Fig. 8 is an explanatory view showing an example of the observation image at steps S4 and S6 of Fig. 5;
Fig. 9 is an explanatory view showing an example of the observation image at a step S8 of Fig. 5 in which a lesion remains even after laser irradiation;
Fig. 10 is an explanatory view showing an example of the observation image at the step S8 in which the lesion has disappeared;
Fig. 11 is a schematic view of the monitor screen that displays a warning window at a step S10 of Fig. 5;

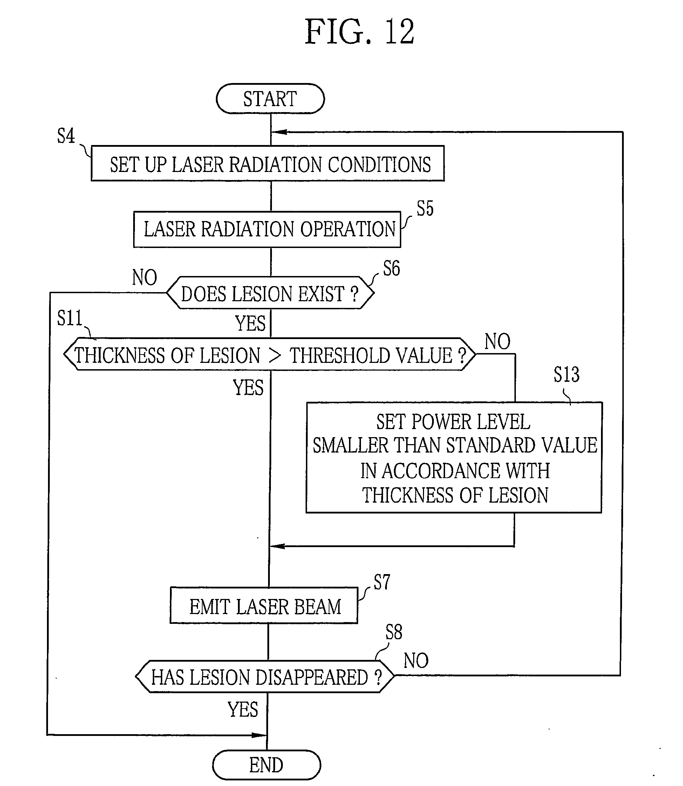

Fig. 12 is a flowchart of a second embodiment in which a power level of laser radiation is varied in response to the degree of disappearance of a lesion;
Fig. 13 is an explanatory view of a tomographic image in which a circular treatment area designation line is displayed;
Fig. 14 is a flowchart of a third embodiment in which a treatment area is designated in a tomographic image;
Fig. 15 is an explanatory view of designating the treatment area in the tomographic image;
Fig. 16 is a flowchart of another embodiment in an automatic mode;
Fig. 17 is an explanatory view of a three-dimensional image in which the treatment area is designated; and
Fig. 18 is a perspective view of a laser treatment apparatus according to further another embodiment.

### DESCRIPTION OF THE PREFERRED EMBODIMENTS

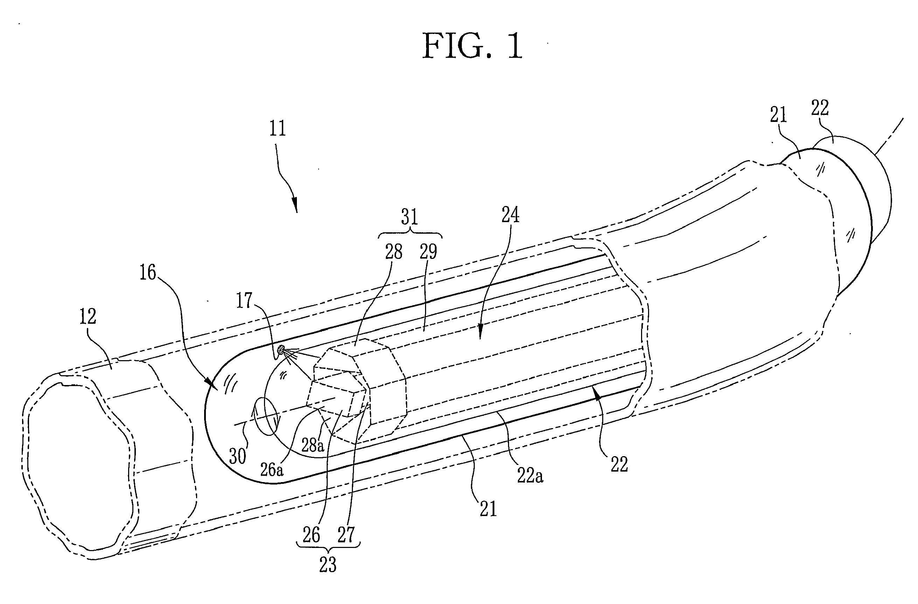

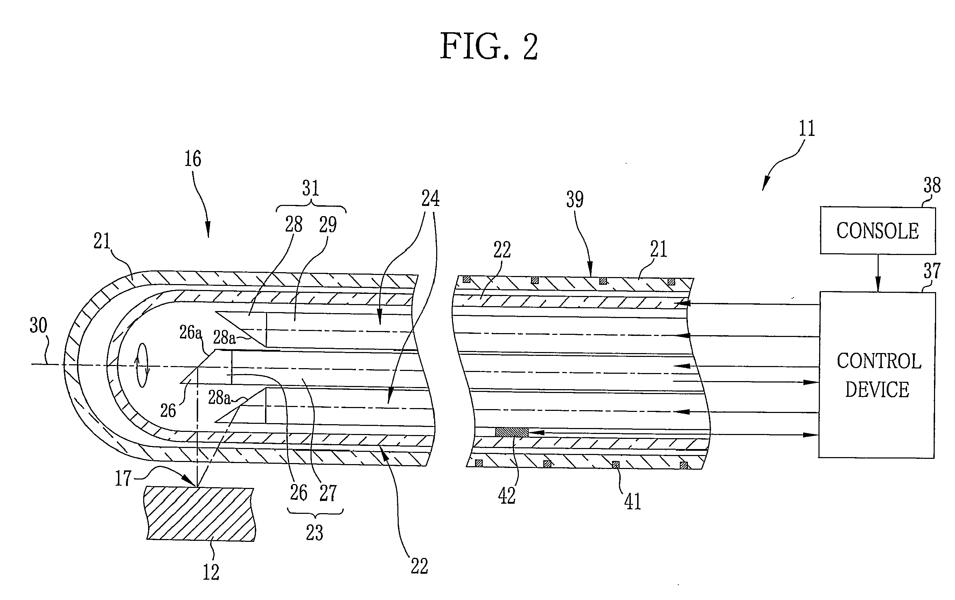

In Figs. 1 and 2, a laser treatment apparatus 11 for laser angioplasty is provided with an insert assembly or catheter assembly 16 that is introduced into a blood vessel 12 being a treatment area of a coronary artery or the like. The laser treatment apparatus 11 applies a laser beam to a lesion 17 of stenosis with plaque to remove unwanted tissue from an inner wall of the blood vessel 12 while capturing a tomographic image of the blood vessel 12 via the catheter assembly 16.

The catheter assembly 16 is constituted of a catheter 21 and a probe unit 22. The transparent and flexible catheter 21 forms an outline of the catheter assembly 16. Into the catheter 21, the probe unit 22 is inserted. The catheter assembly 16 flexibly bends inside the blood vessel 12 in accordance with the tortuous shape.

The probe unit 22 has a tube 22a, an observation probe (imaging probe) 23, and a treatment probe 24. When the probe unit 22 is bent along and swept in the catheter 21, the tube 22a, the observation probe 23, and the treatment probe 24 are integrally bent and swept. The tube 22a is made of transparent and flexible material as with the catheter 21.

To capture the tomographic image, the observation probe 23 being the so-called OCT probe applies low-coherent light to an inner wall of the blood vessel 12, and leads reflected light from the inner wall to a control device 37.

The observation probe 23 has a reflective optical member (optics) 26 and a light guide 27. The light guide 27 is composed of an optical fiber, a GRIN lens, and the like, and is flexibly bent along curves of the probe unit 22. The light guide 27 leads the low-coherent light into the reflective optical member 26, and leads the reflected light from the inner wall of the blood vessel 12 to the control device 37. The reflective optical member 26, which consists of, for example, a prism, is provided at a tip of the light guide 27. A reflective surface 26a of the reflective optical member 26 bends a path of the low-coherent light incident from the light guide 27 in a radial direction of the tube 22a, and applies the low-coherent light to the inner wall of the blood vessel 12. The reflective surface 26a bends the reflected light from the inner wall by 90 degrees to lead the reflected light into the light guide 27.

The observation probe 23 rotates about its center line 30 in one direction. The observation probe 23 scans the whole periphery of the inner wall of the blood vessel 12 by applying the low-coherent light while rotating. Then, the observation probe 23 receives the reflected light (tomographic image data) and obtains a tomographic image of the blood vessel 12 in that scanning position. As the tube 22a sweeps inside the catheter 21, the observation probe 23 scans the blood vessel 12 while shifting in an axial direction. A shift of the observation probe 23 produces a lot of tomographic images in different scanning positions in the axial direction.

Irradiation with the laser beam from the treatment probe 24 ablates the lesion 17 to disappear by decomposition or vaporization. The treatment probe 24 includes a plurality of laser emission sections 31. Each laser emission section 31 consists of a reflective optical member (optics) 28 and a light guide 29. The laser emission sections 31 surround the light guide 27 of the observation probe 23. One of the laser emission sections 31 is chosen on the basis of the position of the treatment area, and applies the laser beam to the lesion 17.

As with the light guide 27 of the observation probe 23, the light guide 29 is composed of an optical fiber, a GRIN lens, and the like. The light guide 29 is flexibly bent along curves of the tube 22a, and leads the laser beam into the reflective optical member 28. The reflective optical member 28 is a prism, as with the reflective optical member 26, and is disposed at a tip of the light guide 29. The laser beam led through the light guide 29 is bent by a reflective surface 28a of the reflective optical member 28, and is incident upon the lesion 17. The reflective optical members 28 are positioned nearer to a baser end than the reflective optical member 26 in the axial direction so that optical paths of the reflective optical members 28 do not overlap with that of the reflective optical member 26 of the observation probe 23. The positions of the reflective optical member 26 and the reflective optical members 28 deviate from each other in the axial direction. However, the reflective surface 26a of the reflective optical member 26 has a different tilt angle from the reflective surface 28a of the reflective optical members 28, so that the low-coherent light and the laser beam are incident on the same position in the axial direction (longitudinal direction) of the blood vessel 12.

The treatment probe 24 has eight laser emission sections 31, but may have more laser emission sections 31 so as to apply the laser beam to an arbitrary position of the inner wall.

As shown in Fig. 2, the laser treatment apparatus 11 is provided with the control device 37 and a console 38. The catheter assembly 16 is connected to the control device 37 and the like.

The low-coherent light is led from the control device 37 into the observation probe 23. The observation probe 23 applies the low-coherent light to the blood vessel 12. The observation probe 23 receives the reflected light, and leads the reflected light into the control device 37. The observation probe 23 is rotated by a motor 40 about the center line 30 at a constant speed. The rotation of the motor 40 is controlled by the control device 37. The motor 40 may be provided in a radiation source unit 43.

The wavelength of the laser light is determined in accordance with the type of the lesion 17 and therapeutic strategy, that is, whether to vaporize the lesion 17 or decompose it. The control device 37 outputs the laser beam with an appropriate wavelength, and inputs the laser beam to the treatment probe 24. The power level of the laser beam is adjusted according to the size and depth of the lesion 17. The wavelength and the power level of the laser beam may be varied by, for example, a surgeon or cardiologist inputting appropriate values to the control device 37 from the console 38. Otherwise, when the laser treatment apparatus 11 is set in an automatic mode of an assistance mode, the control device 37 automatically chooses the appropriate wavelength and power level.

At a base end 39 of the catheter assembly 16, are provided markers 41 and a photo sensor 42 for detecting the markers 41. The annular markers 41 are printed on the periphery of the catheter 21 at regular intervals along the center line 30. The photo sensor 42 detects the markers 41 when the probe unit 22 shifts within the catheter 21. The photo sensor 42 inputs a detection signal of the markers 41 to the control device 37. The detection signal is used for controlling the axial position of the probe unit 22 in the catheter 21.

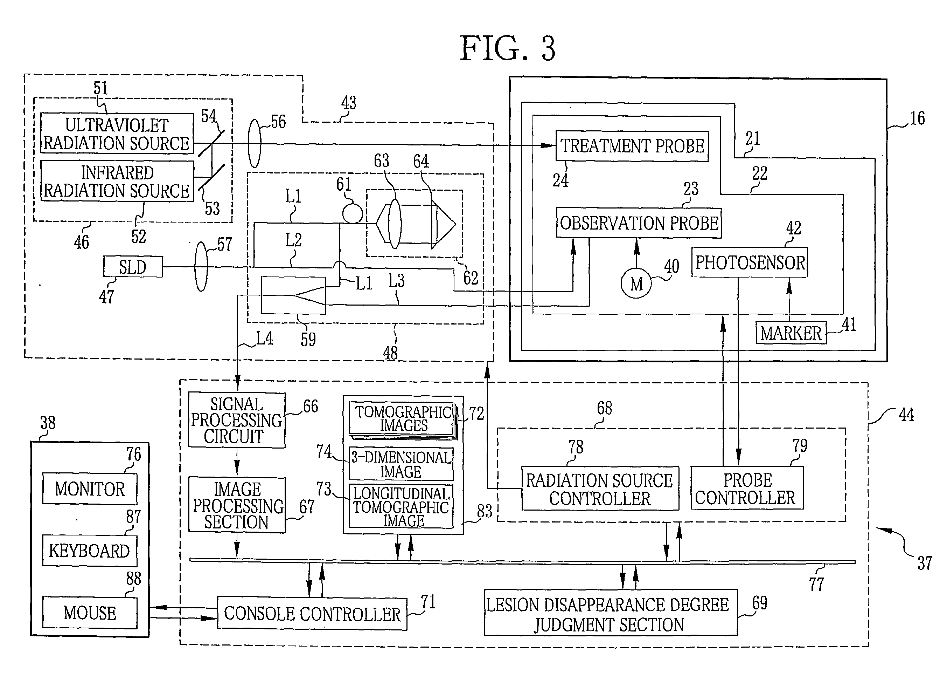

Referring to Fig. 3, the control device 37 includes a radiation source unit 43 and a main unit 44. The radiation source unit 43 has a laser source section 46, super-luminescent diode (SLD) 47, and a fiber coupling optical system 48, and the like.

The laser source section 46 is provided with, for example, an ultraviolet radiation source 51 and an infrared radiation source 52. The ultraviolet radiation source 51 is an excimer laser using XeCl, and outputs a pulse beam of an ultraviolet laser with a wavelength of 308 nm. The ultraviolet laser beam with a wavelength of 308 nm decomposes plaque by photoacoustic process and photochemical process. Accordingly, the ultraviolet radiation source 51 is used for selectively decomposing the plaque without thermally damaging normal tissue.

The infrared radiation source 52 is quantum-cascade laser, and outputs a pulse beam of an infrared laser with a wavelength of 5.75 µm. It is known in the art of medical laser that the infrared laser beam with a wavelength of 5.75 µm is selectively absorbed into an ester bond of cholesterol ester, and splits the ester bond. Thus, the infrared radiation source 52 is used for decomposing lipid plaque that is predominantly composed of the cholesterol ester. Irradiation with the infrared laser beam with a wavelength of 5.75 µm at a moderate power level selectively decomposes the lipid plaque, and moreover, irradiation therewith at a high power level vaporizes peripheral tissue including the lipid plaque. Thus, the infrared radiation source 52 applies the infrared laser beam at the high power level to vaporize the plaque. The output of the infrared radiation source 52 is controlled by a radiation source controller 78.

A reflective mirror 53 and a semi-transparent mirror 54 are disposed in beam paths of the ultraviolet radiation source 51 and the infrared radiation source 52. The reflective mirror 53 reflects infrared rays. The semi-transparent mirror 54 reflects the infrared rays and passes ultraviolet rays. Therefore, the infrared laser beam from the infrared radiation source 52 and the ultraviolet laser beam from the ultraviolet radiation source 51 travel in the same optical path. The laser beam outputted from the laser source section 46 is condensed by a lens 56, and propagates through an optical fiber into the treatment probe 24. A selector (not illustrated) is disposed between the optical fiber and the eight laser emitting sections 31, and the laser beam is incident on the selected one laser emitting section 31.

The SLD 47, being a low-coherent light source, outputs the low-coherent light with a center wavelength of 800 nm and a coherent light of 2 µm. The low-coherent light from the SLD 47 is condensed by a lens 57, and propagates through an optical fiber into the fiber coupling optical system 48.

The fiber coupling optical system 48 is composed of a fiber coupler 59, a piezoelectric element 61, an optical delay section 62, and the like. The low-coherent light from the SLD 47 is split into reference light L1 and signal light L2 by branching off the optical fiber. The reference light L1 is incident on the optical delay section 62. The signal light L2 is incident on the inner wall of the blood vessel 12 via the observation probe 23. The signal light L2 is reflected at various depths of the inner wall of the blood vessel 12, and returns to the fiber coupling optical system 48 as reflected light L3. The reflected light L3 is a mixture of reflected light from the various depths of the inner wall of the blood vessel 12, and is used for obtaining the tomographic image of the blood vessel 12.

The piezoelectric element 61 is disposed in an optical path (optical fiber) that leads the reference light L1 to the optical delay section 62. The piezoelectric element 61 modulates the reference light L1 so as to have a slight frequency difference (Δf from the signal light L3. The optical delay section 62 is composed of a lens 63 and a prism 64 that is movable along the optical axial of the lens 63. In the optical delay section 62, the reference light L1 is converted into parallel rays by the lens 63, and is incident on the prism 64. The reference light L1 reflected from the prism 64 is condensed by the lens 63, and then propagates in a direction opposite to incident light back to the optical fiber. At this time, moving the prism 64 along the optical axis of the lens 63 varies the optical path length of the reference light L1.

The reference light L1 enters the fiber coupler 59 together with the reflected signal light L3. In the fiber coupler 59, the reference light L1 and the reflected signal light L3 are superimposed and become interference light L4. The interference light L4 is inputted to a signal processing circuit 66.

Since the reference light L1 and the reflected signal light L3 is the low-coherent light, when the optical path length of the reference light L1 is equal to the total optical path length of the signal light L2 and the reflected signal light L3, a beat signal that becomes repeatedly stronger and weaker at an interval of the frequency difference (Δf) occurs in the interference light L4. The signal processing circuit 66 detects the strength of the reflected signal light L3 reflected from a predetermined depth of the inner wall, and obtains tomographic data at that depth. Accordingly, moving the prism 64 and varying the optical path length of the reference light L1 vary the optical path length of the reflected signal light L3 interfering therewith, and hence make it possible to obtain the tomographic data at various depths. Furthermore, while the signal light L2 scans the entire periphery of the inner wall of the blood vessel 12, the prism 64 is shifted to vary imaging depth. Therefore, it is possible to obtain the tomographic image from a surface to some depth of the inner wall of the blood vessel 12.

The main unit 44 is composed of the signal processing circuit 66, an image processing section 67, a CPU 68, a lesion disappearance degree judgment section 69 or absence detector, a console controller 71, a RAM 83, and a system bus 77.

The signal processing circuit 66 includes a photodetector and a heterodyne detector. The signal processing circuit 66 converts the strength of the interference light L4 into an electric signal. When the prism 64 moves and the observation probe 23 rotates, the signal processing circuit 66 detects a signal for obtaining the tomographic image from the continuously inputted interference light L4, and outputs that signal to the image processing section 67.

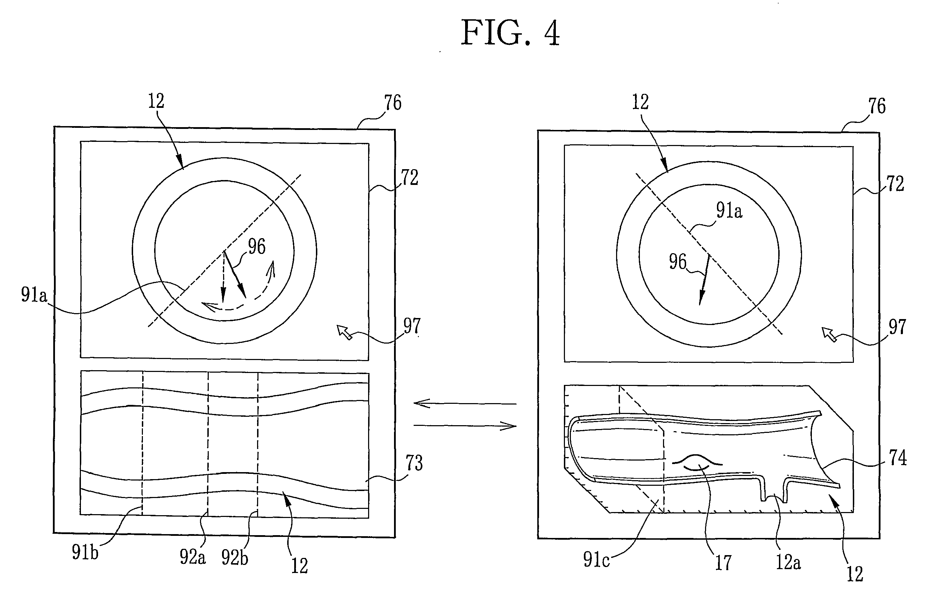

The image processing section 67 produces a tomographic image 72 of the blood vessel 12, as shown in Fig. 4, from the signal successively inputted from the signal processing circuit 66. The tomographic image 72 is a sectional image of the blood vessel 12 orthogonal to a vessel axial direction. The tomographic images 72 at various positions of the blood vessel 12 are obtained with a sweep of the probe unit 22 in the catheter 21. The image processing section 67 also generates a tomographic image 73 or B-scan image (hereinafter called parallel tomographic image) of the blood vessel 12 in a direction parallel to its vessel axis and a three-dimensional image 74 (hereinafter called 3D image) of the blood vessel 12 from a plurality of tomographic images 72 produced by a single sweep of the probe unit 22. The tomographic images 72, the parallel tomographic image 73, and the 3D image 74 (hereinafter collectively called observation images) produced by the image processing section 67 are stored on a RAM 83, and displayed on a screen of a monitor 76. In the tomographic images 72 and the parallel tomographic image 73, difference in tissue manifests itself in difference in luminance. Thus, it is possible to observe the state of normal tissue of a tunica intima, media, and adventitia of the blood vessel 12 and the state of the lesion 17 such as plaque by the distribution of luminance.

The CPU 68 is connected to the image processing section 67, the lesion disappearance degree judgment section 69, the console controller 71, and the like via the system bus 77 to control the operation thereof. The CPU 68 also monitors whether or not the lesion treatment apparatus 11 is appropriately operated based on a judgment result of the lesion disappearance degree judgment section 69 to be described later on. When the laser treatment apparatus 11 has been operated inappropriately, the CPU 68 displays a warning that says operation is inappropriate or a guide for appropriate use on the screen of the monitor 76. The CPU 68 is provided with the radiation source controller 78 and a probe controller 79.

The radiation source controller 78 is connected to the radiation source unit 43. The radiation source controller 78 turns on and off the laser source section 46 and the SLD 47. The radiation source controller 78 switches and adjusts the radiation conditions such as the wavelength and the power level of the laser beam. Fog example, the radiation source controller 78 selectively drives one of the ultraviolet radiation source 51 and the infrared radiation source 52, and switches the wavelength of the laser beam outputted from the laser source section 46. The radiation source controller 78 adjusts an amount of radiation (power level) of the laser beam outputted from the laser source section 46 per unit of time according to therapeutic strategy for laser ablation such as whether to vaporize or decompose the plaque, the thickness of the lesion 17, and the like. The adjustment of the power level is carried out by varying at least one of the pulse frequency, the pulse width, and the duty ratio of the ultraviolet radiation source 51 of the infrared radiation source 52. Furthermore, the radiation source controller 78 turns on and off the SLD 47 and controls the sweeping speed of the prism 64.

When the surgeon or cardiologist has set up the laser radiation conditions and the laser beam has been applied, the radiation source controller 78 judges whether or not the laser radiation conditions set by the surgeon area appropriate based on the judgment result of the lesion disappearance degree judgment section 69. As a result of judgment, when the laser radiation conditions are inappropriate, a warning is displayed on the screen of the monitor 76 via the console controller 71, and the laser source section 46 is forcefully turned off. The laser radiation conditions may be checked before applying the laser beam.

The probe controller 79 is connected to each part of the probe unit 22 and controls the operation thereof. The probe controller 79 controls the rotation of the motor 40 so that the tomographic images 72 of the blood vessel 12 are obtained at constant intervals. The probe controller 79 is connected to a drive system (not illustrated) that shifts the probe unit 22 within the catheter 21. When the laser treatment apparatus 11 is in the automatic mode, the probe controller 79 controls the shift of the probe unit 22 within the catheter 21 in accordance with the size of the lesion 17 in the longitudinal direction of the blood vessel 12. At this time, compensating a drive and a backlash of the drive system, which drives the probe unit 22 based on a signal from the photosensor 42, results in precise control of the position of the probe unit 22 with respect to the catheter 21.

When the surgeon manually shifts the probe unit 22, the probe controller 79 monitors the position of the probe unit 22 within the catheter 21. At this time, the axial shift range of the probe unit 22 is regulated in order to prevent damage of the catheter 21 by inserting the probe unit 22 too deeply and damage of the probe unit 22 by being pulled out of the catheter 21. When the probe unit 22 almost goes out of the shift range, the probe controller 79 displays a warning on the screen of the monitor 76 through the console controller 71 and forcefully blocks the shift of the probe unit 22.

The lesion disappearance degree judgment section 69 reads the observation images such as the tomographic images 72 generated by the image processing section 67 out of the RAM 83, and distinguishes the lesion 17 by analyzing the distribution of luminance of the observation images. Accordingly, the presence or absence of the lesion 17 and the size of the lesion 17 such as thickness, angular range (in the tomographic images 72, the size of the lesion 17 indicated by a central angle), and length are determined. The lesion disappearance degree judgment section 69 checks the presence or absence of the lesion 17 on the tomographic images 72 that are newly obtained after laser beam irradiation in order to judge whether or not the lesion 17 has disappeared (the degree of disappearance) by the laser beam irradiation. A judgment result of the lesion disappearance degree judgment section 69 is temporarily stored on the RAM 83, and is used by the CPU 68 for controlling the operation of the laser treatment apparatus 11.

The console controller 71 controls the console 38. The console 38 includes the monitor 76 for displaying the observation images and operation menus and an input device such as a keyboard 87 and a mouse 88. Whenever the laser treatment apparatus 11 obtains the tomographic image 72, the console controller 71 immediately reads the tomographic image 72 from the RAM 83 and displays the tomographic image 72 on the screen of the monitor 76. Accordingly, the moving tomographic images 72 are displayed on the screen of the monitor 76.

Together with the observation image, the console controller 71 displays on the monitor 76 a menu for guiding the operation of the laser treatment apparatus 11 and a tool for designating a treatment area in the observation image. When a plurality of observation images are displayed on the screen of the monitor 76, for example, reference lines 91a, 91b, and 91c are displayed to show correspondence among the tomographic image 72, the parallel tomographic image 73, and the 3D image 74. In applying the laser beam, an arrow 96 or the like is displayed in the tomographic image 72 to show a laser beam emitting direction. The surgeon operates the menu and the tool (operation tool by a GUI) displayed on the monitor 76 with the use of the keyboard 87 and the mouse 88.

The RAM 83 is a memory for storing the tomographic images 72 and various setting values. The RAM 83 stores various settings, data, and the like such as the power level (hereinafter called standard value) of the laser beam applied to a relatively large lesion 17, a threshold value compared with the axial size (thickness) of the lesion 17, and the luminance value of the lesion 17 designated by the surgeon, in addition to the observation images.

The laser treatment apparatus 11 with the foregoing structure has three operation modes, that is, a manual mode, the assistance mode, and the automatic mode. The laser treatment apparatus 11 is switchable into an arbitrary operation mode according to the therapeutic strategy and the type of lesion 17.

In the manual mode, the surgeon manually operates the entire functions of the laser treatment apparatus 11. The surgeon also carried out a sweep of the probe unit 22, designation of the treatment area, and a setup of the laser radiation conditions such as the wavelength and the power level of the laser beam during therapy.

In the assistance mode, although the surgeon manually operates some of the functions of the laser treatment apparatus 11, the laser treatment apparatus 11 automatically carried out complicated operation and settings. When the laser treatment apparatus 11 is operated inappropriately, a warning or a guide is displayed to assist the surgeon.

In the automatic mode, the laser treatment apparatus 11 automatically carries out the entire operation after inserting the catheter assembly 16 into the blood vessel 12. For example, when the surgeon orders to start obtaining the tomographic images 72 by the console 38, the laser treatment apparatus 11 automatically sweeps the probe unit 22 and obtains the tomographic image 72. After designation of the treatment area, when the surgeon orders to start applying the laser beam, the laser treatment apparatus 11 automatically recognizes the lesion 17 within the designated treatment area (angular area and longitudinal area), and irradiates the lesion 17 with the laser beam.

### [First Embodiment]

In the following first to third embodiments, the laser treatment apparatus 11 is set in the assistance mode. In the first embodiment, the degree of disappearance of the lesion 17 is judged whenever the laser beam has been applied for a predetermined time, and the laser source section 46 is turned on and off in response to a judgment result. When the catheter assembly 16 is introduced into the blood vessel 12, the probe unit 22 sweeps and the tomographic images 72 of the blood vessel 12 are produced from the signal of the observation probe 23.

The tomographic image 72 is displayed on the monitor 76 in real time, as shown in Fig. 4. The parallel tomographic image 73 and the 3D image 74 are produced from the obtained plural tomographic images 72. One of the parallel tomographic image 73 (left of Fig. 4) and the 3D image 74 (right of Fig. 4) selected by the console 38 is displayed on the screen of the monitor 76 together with the tomographic image 72.

As shown in' left of Fig. 4, the reference line 91a is displayed in the tomographic image 72, and the reference line 91b is displayed in the parallel tomographic image 73. The reference lines 91a and 91b indicate the positional relation between the tomographic image 72 and the parallel tomographic image 73. The reference lien 91a is rotatable and movable, and the reference line 91b is movable. When the surgeon or cardiologist moves the reference line 91a or 91b by dragging a cursor or mouse pointer 97, the parallel tomographic image 73 or the tomographic image 72 corresponding to the position of the moved reference line 91a or 91b is newly displayed on the monitor 76. To be more specific, when the reference line 91a is rotated in the tomographic image 72, the displayed parallel tomographic image 73 is switched into a new one that corresponds to a section taken along the reference line 91a after the rotation. In a like manner, when the reference line 91b is moved to the right or left in the parallel tomographic image 73, the displayed tomographic image 72 is switched into a new one that corresponds to the position of the reference line 91b after the movement.

A pair of treatment area designation lines 92a and 92b is displayed in the parallel tomographic image 73. The surgeon shifts the treatment area designation lines 92a and 92b in the vessel axial direction by the cursor 97 so that the lesion 17 is contained in an area between the lines 92a and 92b. The area between the lines 92a and 92b that contains the lesion 17 is designated as the treatment area. Within the treatment area designated like this, the probe unit 22 shifts in the catheter 21 to obtain the observation images, to irradiate the lesion 17 with the laser beam, and to judge the degree of disappearance of the lesion 17.

In displaying the 3D image 74 together with the tomographic image 72, as shown in right of Fig. 4, the 3D image 74 of the blood vessel 12 sectioned along the reference line 91a is displayed below the tomographic image 72. At this time, the reference line 91c is displayed in the 3D image 74 to indicate a position corresponding to the tomographic image 72. As in the case of the parallel tomographic image 73, when the reference line 91c is shifted, the displayed tomographic image 72 is updated to a new one that corresponds to the position of the reference line 91c after the shift. Rotating the reference line 91a in the tomographic image 72 changes the sectional direction of the blood vessel 12 in the 3D image 74. The 3D image 74 is displayed on the monitor 76 in such a direction as to display the inner wall. Since the 3D image 74 appears to display the blood vessel 12 in three dimension, it becomes relatively easy for the surgeon to find out the lesion 17 or a branch 12a of the blood vessel 12, which could not be found out in the parallel tomographic image 73 without adjusting the reference lines 91a and 91b.

In the tomographic image 72, the arrow 96 that shows the laser beam emitting direction is displayed. In applying the laser beam, the surgeon or cardiologist rotates the arrow 96, and determines the laser beam emitting direction so as to correctly irradiate the lesion 17 with the laser beam.

When the observation images are displayed on the monitor 76, the surgeon grasps the presence or absence of the lesion 17 and the state thereof while watching the observation images. The surgeon irradiates the lesion 17 with the laser beam while monitoring the process of disappearance of the lesion 17 in real time on the tomographic image 72.

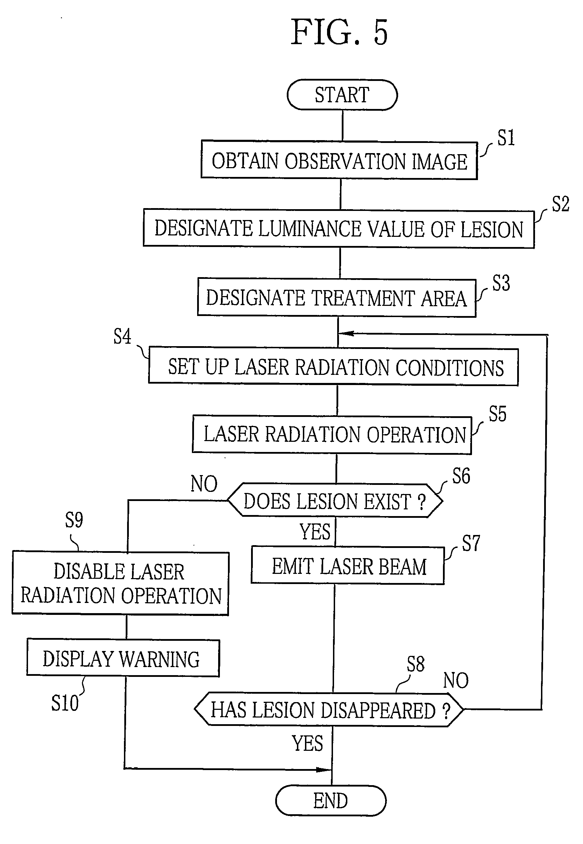

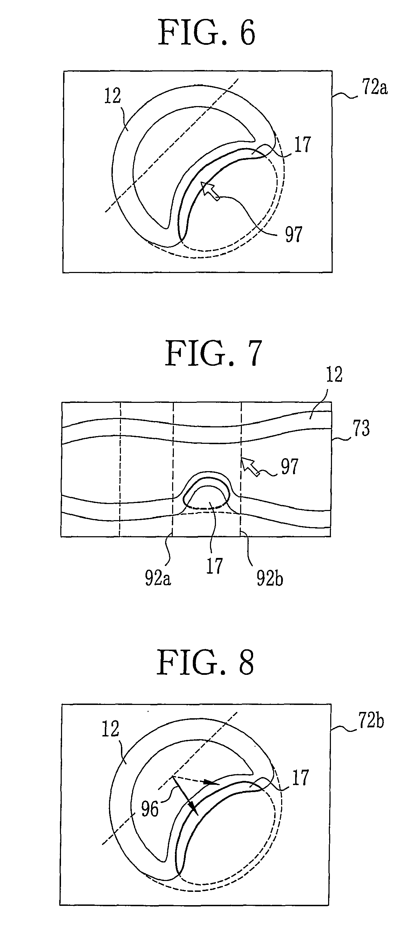

As shown in Fig. 5, the observation images are first obtained (step S1). The surgeon displays on the monitor 76 the tomographic image 72a (Fig. 6) with the lesion 17. When the surgeon moves the cursor 97 over the lesion 17 and clicks the mouse 88, the luminance value of the lesion 17 is designated (step S2). The luminance value is a reference value by which the lesion disappearance degree judgment section 69 distinguishes the lesion 17 by image analysis. The laser treatment apparatus 11 recognizes an area having the designated luminance value as the lesion 17 in the tomographic image 72a.

The surgeon encloses the lesion 17 with the treatment area designation lines 92a and 92b while watching the parallel tomographic image 73 (or the 3D image 74) (Fig. 7). The surgeon sets a treatment area neither too much nor too little in such a manner that the length of the treatment area in the vessel axial direction of the blood vessel 12 is approximately equal to the length of the lesion 17 (step S3). Treatment area data in the vessel axial direction is inputted to the CPU 68 via the console 38. Once the treatment area is designated, the probe unit 22 is allowed to sweep only within the treatment area, and laser beam irradiation is allowed only within the treatment area. Upon completing the designation of the treatment area, the probe unit 22 is shifted to a treatment start position at an end of the treatment area. The observation probe 23 keeps capturing the tomographic images 72 while doing so, an updated tomographic image 72 is displayed on the monitor 76.

When the probe unit 22 is shifted to the treatment start position, the surgeon sets up the laser radiation conditions, that is, a choice of the used laser source, the wavelength and the power level of the laser beam, and the like in accordance with the size and depth of the lesion 17, while observing the tomographic image 72b (Fig. 8) in the treatment start position (step S4). On the screen of the monitor 76, the arrow 96 that indicates a laser beam emitting direction is displayed in the tomographic image 72b. The surgeon rotates the arrow 96 so as to be aimed at the lesion 17, and hence the laser beam emitting direction is designated.

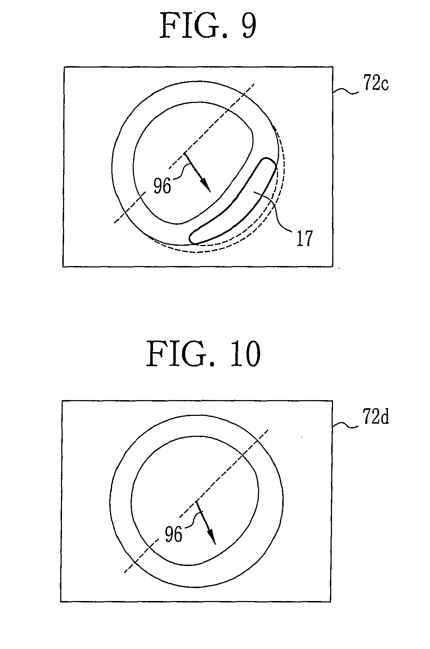

When the laser radiation conditions have been completely set, laser radiation operation is carried out (step S5). The laser treatment apparatus 11 judges whether or not a pixel having the designated luminance value corresponding to the lesion 17 exists in the tomographic image 72b, in other words, whether or not the lesion 17 exists therein (step S6). When the lesion 17 exists in the tomographic image 72b as shown in Fig. 8, the laser treatment apparatus 11 allows the laser beam radiation. The laser source section 46 is turned on, and irradiates the lesion 17 with the laser beam with the designated radiation conditions for the predetermined time (step S7). Then, the laser treatment apparatus 11 judges whether or not a pixel having the designated luminance value still exists in the tomographic image 72c after the laser beam irradiation, in short, the degree of disappearance of the lesion 17 (step S8). At this time, if the lesion remains, the foregoing procedure from step S4 to step S8 is repeated to irradiate the lesion 17 with the laser beam, until the lesion 17 completely disappears as shown in a tomographic image 72d in Fig. 10.

On the other hand, when the lesion 17 does not exist in the designated laser beam emitting direction although the laser radiation operation has been carried out, in other words, when the laser beam emitting direction is inappropriate, the laser treatment apparatus 11 disables the laser radiation operation. The laser source section 46 is forcefully turned off, and cancels new laser radiation operation (step S9). Then, a warning is displayed on the monitor screen (step S10).

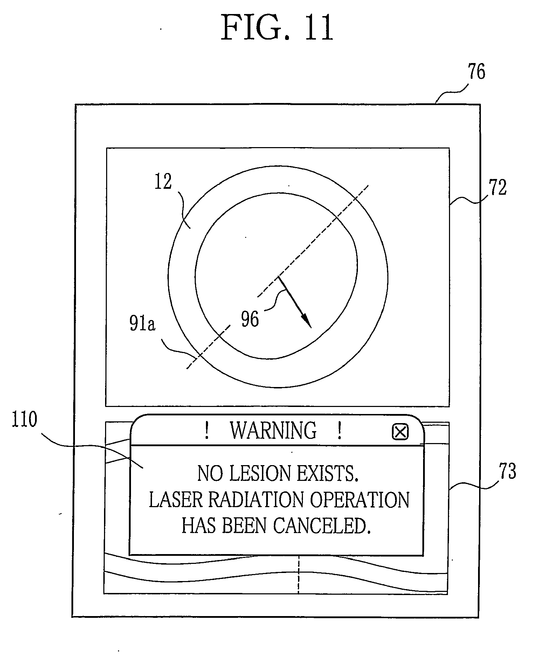

In step S10, a warning window 110 is displayed on the screen of the monitor 76 as shown in Fig. 11. The warning window 110 notifies the surgeon of the disappearance of the lesion 17 and the cancellation of the laser radiation operation. After YES in step S8, a message that says the lesion 17 has disappeared by laser beam irradiation may be displayed on the monitor screen.

After the lesion 17 disappears from a certain position in the treatment area as described above, the probe unit 22 sweeps from the treatment start position in one direction by a certain procedure, and applies the laser beam to a position after the sweep. By repeating the foregoing procedure, the entire treatment area is irradiated with the laser beam.

The laser treatment apparatus 11, as described above, judges the degree of disappearance of the lesion 17 from the tomographic image 72 whenever the laser beam has been emitted. The laser treatment apparatus 11 controls the laser source section 46 based on the judgment result. Accordingly, it is possible to prevent incorrect laser radiation and provide safe therapy.

### [Second Embodiment]

In a second embodiment, the power level of the laser beam is controlled based on the degree of disappearance of the lesion 17. As with the first embodiment, the observation images are obtained (step S1), and the luminance value of the lesion 17 is designated (step S2). Then, the treatment area is designated in the vessel axial direction of the blood vessel 12 (step S3), and the laser radiation conditions are set up (step S4). When the laser radiation operation is carried out (step S5), the laser treatment apparatus 11 judges whether or not a pixel having the designated luminance value exists in the laser tomographic image 72 (step S6).

As a result of judgment, when the lesion 17 exists in the tomographic image 72, the lesion disappearance degree judgment section 69 judges the thickness of the lesion 17 in the designated laser beam emitting direction, and inputs the thickness to the CPU 68. The CPU 68 compares the thickness of the lesion 17 to a predetermined threshold value. When the thickness of the lesion 17 is larger than the threshold value, in other words, the lesion 17 is judged to be relatively thick, the laser beam is emitted with a standard power level. The standard power level s suited for treatment of the relatively thick lesion 17, as described above, and varies from one laser source to another.

When the thickness of the lesion 17 is the threshold value or less, on the other hand, the radiation source controller 78 sets the power level of the laser beam smaller than the standard value in accordance with the thickness of the lesion 17 (step S13), and then the laser beam is emitted (step S7). At this time, the CPU 68 sets the power level of the laser beam in such a manner that the thicker the lesion 17, the nearer the power level is to the standard value, and that the thinner the lesion 17, the smaller the power level is.

After radiation of the laser beam for the predetermined time at the power level corresponding to the thickness of the lesion 17, as in the case of the first embodiment, the degree of disappearance of the lesion 17 is judged from the latest tomographic image 72 (step S8). As a result of this, if the lesion 17 has not disappeared, the foregoing laser beam radiation process is repeated until the lesion 17 disappears. If the lesion 17 has disappeared in the synchronously captured tomographic image 72, on the other hand, the laser beam is not emitted anymore. Then, as in the case of the first embodiment, after the lesion 17 has disappeared from an area irradiated with the laser beam, the probe unit 22 is swept by a predetermined distance in accordance with the size of the laser beam.

Automatically controlling the power level of the laser beam according to the thickness of the lesion 17, as described above, can prevent the small and thin lesion 17 from being irradiated with the laser beam at excessive power level. The laser treatment apparatus 11 has a limit in the penetration in the radial length (tomographic depth) of the blood vessel 12. When the lesion 17 is large and thick, the entire outline of the lesion 17 cannot be imaged on the tomographic image 72. However, automatically adjusting the power level, as described above, is helpful to safely irradiate the thick lesion 17 with the laser beam.

The irradiation position or the wavelength of the laser beam may be switchable according to the degree of disappearance of the lesion 17. For example, the lesion disappearance degree judgment section 69 recognizes an angular range of the lesion 17 with respect to the center line 30 based on the degree of disappearance of the lesion 17, and inputs angular range data to the CPU 68. The CPU 68 may automatically adjust the laser beam emitting direction within the inputted angular range. Otherwise, the lesion disappearance degree judgment section 69 may recognize the thickness of the lesion 17, and input thickness data to the CPU 68. The CPU 68 compares the thickness of the lesion 17 with a threshold value. When the lesion 17 is thicker than the threshold value, the infrared radiation source 52 emits the infrared laser beam at the high power level. When the lesion 17 is thinner than the threshold value, the ultraviolet laser beam from the ultraviolet radiation source 51 is used.

### [Third Embodiment]

In the foregoing first and second embodiments, the treatment area within which the degree of disappearance of the lesion 17 is judged is designated in the vessel axial direction of the blood vessel 12, but a sectional area centered on a vessel axis may be designated as the treatment area.

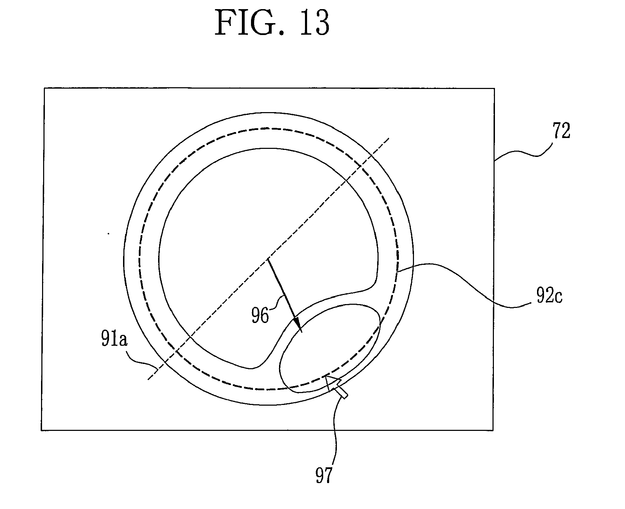

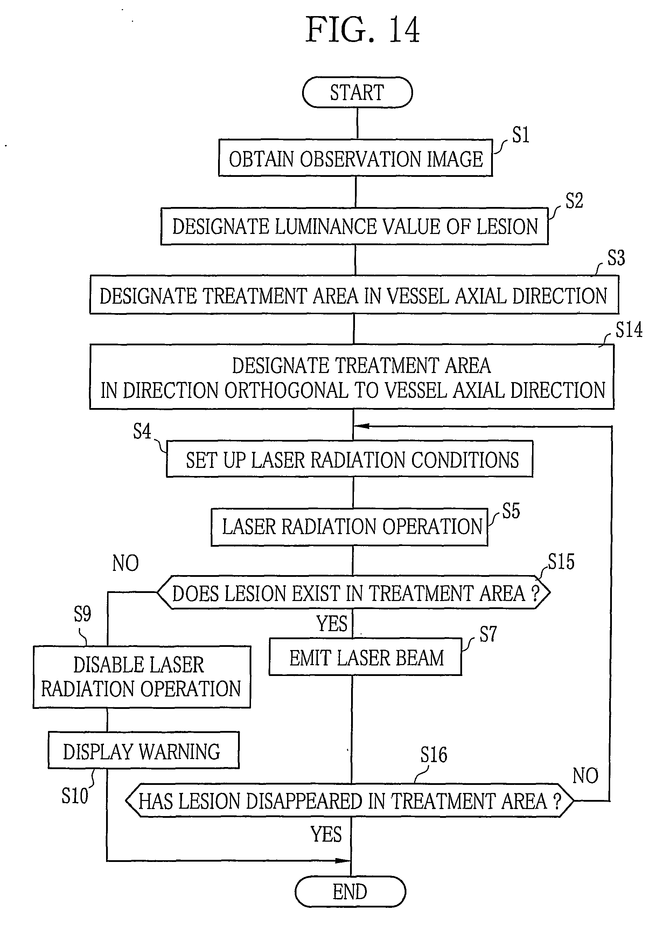

In the third embodiment, as shown in Fig. 14, the observation images are first obtained (step S1), and then the luminance value of the lesion 17 is inputted (step S2). The surgeon or cardiologist encloses the lesion 17 with the treatment area designation lines 92a and 92b while watching the parallel tomographic image 73 (or the 3D image 74) so as to have an approximately equal length to the lesion 17 in the axial direction. The area between the treatment area designation lines 92a and 92b is designated as the treatment area in the axial direction (step S3). Concurrently, the console controller 71, as shown in Fig. 13, displays a circular treatment area designation line 92c that designates an area in a direction orthogonal to the vessel axis in the tomographic image 72. Dragging the cursor 97 varies the diameter of the treatment area designation line 92c centered on the vessel axis with keeping its circular shape. After varying the diameter of the treatment area designation line 92c, the console controller 71 designates an area surrounded by the treatment area designation line 92c as a treatment area in the direction orthogonal to the vessel axis, and inputs the treatment area to the CPU 68. Thus, the treatment area is designated not only in the vessel axial direction but also in the direction orthogonal to the vessel axis (step S14).

After that, the laser radiation conditions are set up (step S4), and the laser radiation operation is carried out (step S5). At this time, the lesion disappearance degree judgment section 69 judges the degree of disappearance of the lesion 17 not in the entire tomographic image 72 but in the treatment area designated by the treatment area designation line 92c, and inputs a judgment result to the CPU 68 (step S15). The CPU 68 permits the laser radiation operation when the lesion 17 exists within the treatment area, and emits the laser beam for a predetermined time (step S7) . The lesion disappearance degree judgment section 69 judges from the tomographic image 72 after laser beam irradiation whether or not the lesion 17 still exists in the treatment area designated by the treatment area designation line 92c (step S16). The foregoing procedure is repeated until the lesion 17 disappears. If the lesion 17 is judged to be absent in the laser beam emitting direction, the laser radiation operation is disabled, and the laser source section 46 is forcefully turned off (step S9). Then, it is banned to carry out laser radiation operation again, and a warning is displayed (step S10).

After the lesion 17 has disappeared by the laser beam irradiation, the adjoining laser emitting section 31 is chosen and turned on to apply the laser beam to an adjoining area. Repeating the foregoing steps makes the lesion 17 disappear from the entire treatment area. A plurality of laser emitting sections 31 opposite to one another may be simultaneously chosen and apply the laser beams to the lesion 17 at a time.

As described above, the degree of disappearance of the lesion 17 is judged in the treatment area designated in a plane of the tomographic image 72 orthogonal to the vessel axis of the blood vessel 12. The laser source section 46 is turned on and off according to a judgment result. Accordingly, it is possible to ensure the thickness of the blood vessel 12 after laser beam irradiation, and hence safely provide the therapy. This function of the laser treatment apparatus 11 is especially useful in using an infrared laser at the high power level to vaporize the lesion 17.

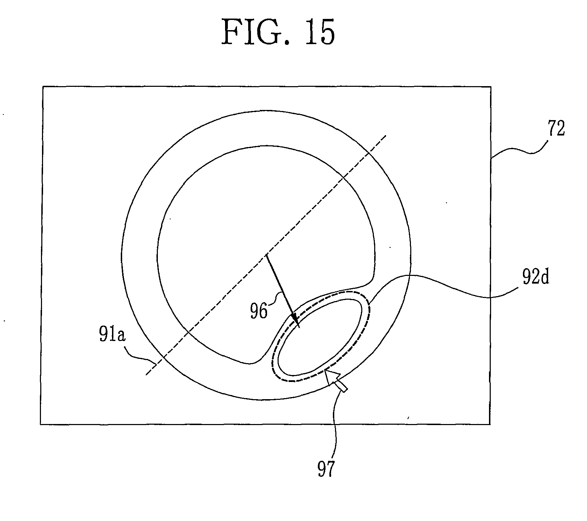

In the third embodiment, only the diameter of the circular treatment area designation line 92c is variable about the vessel axis. However, as shown in Fig. 15, a treatment area designation line 92d the center and shape of which are flexibly variable may be used instead. By moving and deforming the treatment area designation line 92d by the cursor 97, the lesion 17 is enclosed fully with a high degree of freedom so that an area within the treatment area designation line 92d is designated as a treatment area in a plane orthogonal to the vessel axis. When the treatment area is designated and the lesion 17 is vaporized by laser ablation, the entire treatment area may be recognized as the lesion 17, instead of distinguishing the lesion 17 by its luminance value. In this case, the degree of disappearance of the lesion 17 may be judged by the presence or absence of tissue in the treatment area, and the laser source section 46 may be turned on and off in accordance with a judgment result.

An area in an angular direction with respect to the vessel axis of the blood vessel 12 may be designated as the treatment area in the tomographic image 72. In this case, for example, two straight lines rotating about the vessel axis may be displayed on the tomographic image 72 as treatment area designation lines, and an angular area between the treatment area designation lines is designated as the angular treatment area. Then, the degree of disappearance of the lesion 17 is judged in the angular treatment area. According to a judgment result, if the lesion 17 exists in the treatment area, laser beam radiation is allowed. Using the angular treatment area can prevent improper irradiation with the laser beam, when the laser beam emitting direction set by the arrow 96 is inappropriate.

The laser treatment apparatus 11 successively judges the degree of disappearance of the lesion 17 with irradiating the lesion 17 with the laser beam. Based on this judgment result, the laser treatment apparatus 11 turns the laser source section 46 on and off and controls the laser beam emitting direction and the power level. Accordingly, it is possible to prevent the improper or excessive radiation of the laser beam and safely provide therapy. The laser treatment apparatus 11 assists the control of the laser beam as described above, and hence reduces surgeon's operation burden.

In the laser treatment apparatus 11, the luminance value of the lesion 17 is directly designated in the tomographic image 72. This means that the difference between normal tissue and the lesion 17 is directly designated in the actually treated blood vessel 12, so that the lesion 17 is distinguished with high precision.

In the first to third embodiments, the degree of disappearance of the lesion 17 is checked at established intervals, and each of the various laser radiation conditions is controlled according to the degree of disappearance. By combining the foregoing first to third embodiments or a part thereof, it is preferable to control the laser radiation conditions according to the degree of disappearance of the lesion 17.

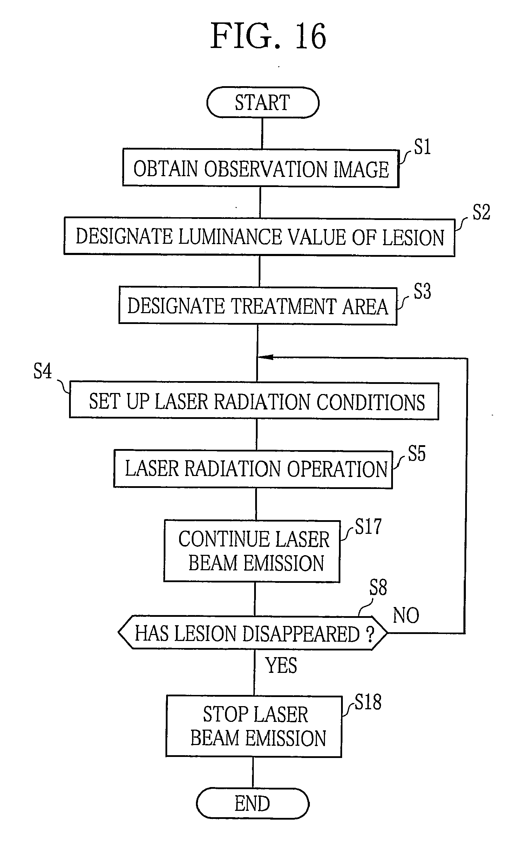

In the first to third embodiments, the laser treatment apparatus 11 is used in the assistance mode. It is preferable that the laser radiation conditions be controlled in a like manner even when the laser treatment apparatus 11 is used in the automatic mode. For example, in the case of the automatic mode shown in Fig. 16, the laser source section 46 is turned on or off based on whether or not the lesion 17 has disappeared (step S8). In the automatic mode, the laser beam is constantly emitted while the lesion 17 exists (step S17). The degree of disappearance of the lesion 17 is checked during the laser radiation operation, to change the laser radiation condition. When the lesion 17 has disappeared, radiation of the laser beam is stopped (step S18). Then, the treatment probe 24 is automatically shifted within the confines of the lesion 17, and the laser emitting section 31 to radiate the laser beam is automatically chosen.

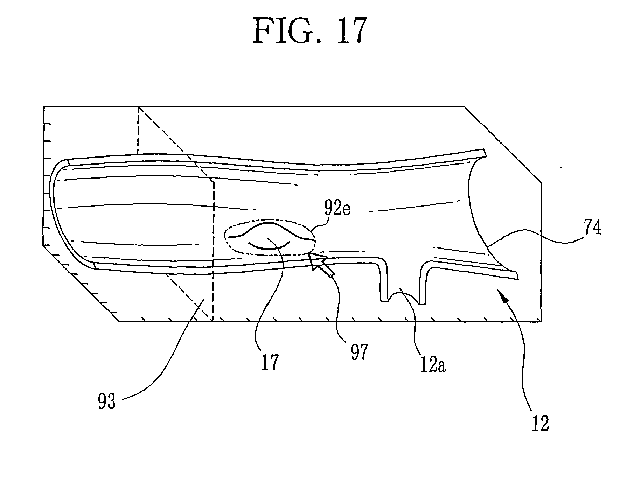

In the first to third embodiments, the treatment area designation lines displayed in the parallel tomographic image 73 and the tomographic image 72 designate the treatment area. The treatment area, however, may be designated in the 3D image 74. For example, when the surgeon clicks on the lesion 17 in the 3D image 74, a surface shape and luminance distribution around an clicked area is analyzed, and a treatment area designation line 92e surrounding the lesion 17 is displayed (refer to Fig. 17). An area within the treatment area designation line 92e may be designated as the treatment area.

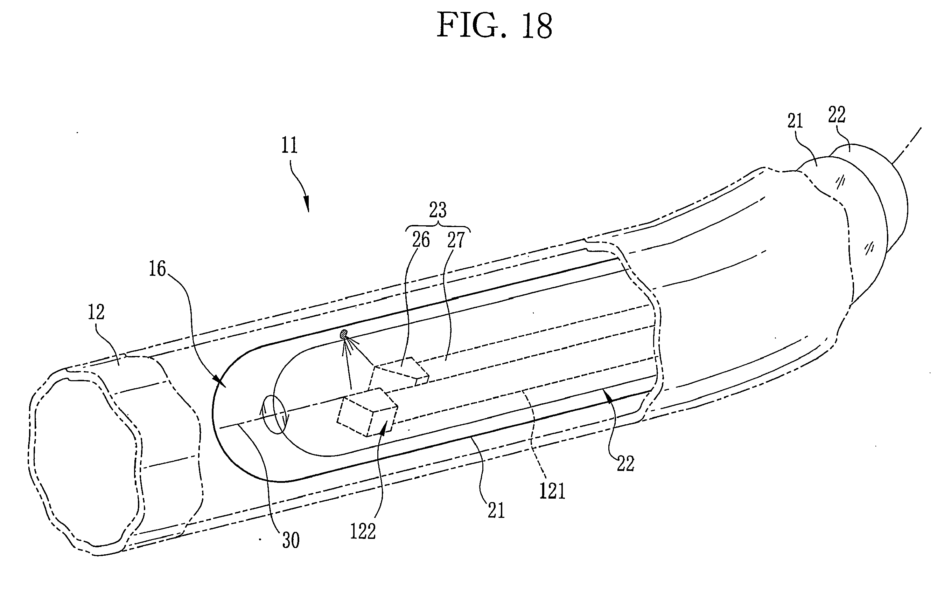

Fig. 18 shows a case where a treatment probe 121 has a single laser emission section 122. The laser emission section 122 rotates together with the observation probe 23. During rotation, the observation probe 23 obtains the tomographic images. The laser emission section 122 is turned on and applies the laser beam only while facing to the lesion 17. The rotation of the laser emission section 122 and the observation probe 23 may be stopped until the lesion 17 irradiated with the laser beam disappears.

In the laser treatment apparatus 11, the console 38 includes the monitor 76, the keyboard 87, and the mouse 88. The console 38 may include a touch panel display having display and input functions instead of or in addition to the above.

In the first to third embodiments, the lesion disappearance degree judgment section 69 judges the position, size, disappearance degree, and the like of the lesion 17 whenever the tomographic image 72 is captured. The lesion disappearance degree judgment section 69 may judge these items at predetermined intervals, irrespective of a capturing cycle of the tomographic image 72.

In the first to third embodiments, the surgeon designates the luminance value of the lesion 17 by operating the console 38. However, difference in luminance between the lesion 17 and normal tissue may be defined and stored in advance. In this case, the lesion 17 is distinguished by judging the difference in luminance. Otherwise, the surgeon or cardiologist may directly input a value on the keyboard 87, or the value may be obtained over a communication network.

As the ultraviolet radiation source 51, an ArF excimer laser with a wavelength of 193 nm, a KrF excimer laser with a wavelength of 248 nm, a XeF excimer laser with a wavelength of 351 nm, or the like is available in addition to the XeCl excimer laser with a wavelength of 308 nm. It is preferable that at least one of these lasers be provided as the ultraviolet radiation source 51. As the infrared radiation source 52, a free electron laser that outputs an infrared laser beam with a wavelength of 5.75 µm or a commonly known semiconductor laser is available in addition to the quantum-cascade laser with a wavelength of 5.75 µm.

In the first to third embodiments, the quantum-cascade laser that selectively emits an infrared laser beam with a wavelength of 5.75 µm and a laser beam with a short wavelength is used as the infrared radiation source 52. It is preferable to provide at least one infrared radiation source 52 that can selectively decompose lipid plaque like this. It is preferable that the infrared radiation source 52 output a laser beam with a short wavelength between or equal to 1100 nm and 1800 nm, in addition to a laser beam with a long wavelength. It is more preferable that at least one infrared radiation source 52 emit a laser beam with a wavelength between or equal to 1200 nm and 1250 nm or a wavelength between or equal to 1680 nm and 1800 nm. It is more preferable to provide a plurality of infrared radiation sources 52 and switch among laser beams with many wavelength bands. However, if the wavelength of the outputted laser beam is variable and the laser beam with the foregoing wavelengths is outputted as desired, only on infrared radiation source 52 may be enough.

The observation probe 23 may be an ultrasonic probe that obtains a tomographic image with the use of ultrasound, instead of an OCT probe unit that obtains a tomographic image with the use of interference of light. The observation probe 23 may be a frequency-domain OCT (OFDI) probe or another OCT probe with a commonly known mechanism, instead of the so-called time-domain OCT probe unit.

In the present invention, electromagnetic waves including light and X-rays, ultrasound, magnetic resonance and other transmission energy in various modalities of medical imaging are available to obtain a tomographic image of the treatment area. The tomographic image is produced by scanning the treatment area in any one of the modalities and processing a detection result of the reflection from the treatment area.

The laser treatment apparatus 11 is also available in percutaneous treatment of another body part than the blood vessel 12 in the human body cavity. For example, the laser treatment apparatus 11 of the invention may be an endoscopic type for treatment of a gastrointestinal tract by oral swallowing.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A laser treatment apparatus (11) having a catheter assembly (16) introduced into a human body cavity, comprising:
a treatment probe (24) disposed in said catheter assembly, for applying a laser beam to a lesion (17) in a treatment area in said body cavity for reaction to material of said lesion;
an observation probe (23) disposed in said catheter assembly together with said treatment probe, for applying electromagnetic radiation (L2) to said treatment area and receiving reflected electromagnetic radiation (L3) from said treatment area;
an image processing section (67) for producing a tomographic image (72) of said treatment area based on said reflected electromagnetic radiation from said observation probe;
a lesion disappearance degree judgment section (69) for analyzing said tomographic image and judging the degree of disappearance of said lesion by said reaction; and
a probe controller (79) for controlling radiation of said laser beam in response to a judgment result of said lesion disappearance degree judgment section.

2. The laser treatment apparatus (11) according to claim 1 further comprising:
a light source (47) for emitting low-coherent light as said electromagnetic radiation;
a light splitter for splitting said low-coherent light into reference light (L1) and signal light (L2), said signal light being incident on said observation probe (23);
a frequency modulator (61) for slightly varying the frequency of said reference light;
a scanner (79) for scanning said treatment area with said signal light by rotating said observation probe, reflected signal light (L3) from said treatment area being incident on said observation probe; and
an interference light generator (59) for generating interference light (L4) by interference of said reflected signal light from said observation probe and said reference light, said image processing section (67) producing said tomographic image (72) from said interference light.

3. The laser treatment apparatus (11) according to claim 2 further comprising:
an optical delay section (62) for varying an optical path length of said reference light (L1) to vary imaging depth thereof.

4. The laser treatment apparatus (11) according to claim 3, wherein
each of said treatment probe (24) and said observation probe (23) has an optical fiber (29, 27) and reflective optical member (28, 26) attached at a tip of said optical fiber; and
said catheter assembly (16) includes a transparent catheter (21).

5. The laser treatment apparatus (11) according to claim 4, wherein
said treatment probe (24) and said observation probe (23) are movable in said catheter (21).

6. The laser treatment apparatus (11) according to claim 1 further comprising:
a monitor (76) for displaying said tomographic image (72).

7. The laser treatment apparatus (11) according to claim 1, wherein said probe controller (79) controls a laser radiation condition that includes at least one of switching radiation of said laser beam, a laser beam irradiation position, an amount of irradiation of said laser beam per unit of time, and a choice of wavelengths of said laser beam.

8. The laser treatment apparatus (11) according to claim 1, wherein the disappearance of said lesion (17) is judged based on luminance of said treatment area by analyzing said tomographic image (72).

9. The laser treatment apparatus (11) according to claim 8, wherein a reference value of luminance of said lesion (17) is obtained, and luminance of said treatment area is compared with said reference value in order to judge the disappearance of said lesion.

10. The laser treatment apparatus (11) according to claim 1, further comprising:
a designation section for designating said treatment area in said tomographic image (72).

11. The laser treatment apparatus (11) according to claim 1, wherein said probe controller (79) has an automatic mode for automatically controlling said treatment probe (24);
in said automatic mode, said treatment probe continues applying said laser beam while said lesion (17) exists, and stops applying said laser beam when said lesion disappearance degree judgment section (69) judges that said lesion has disappeared.

12. The laser treatment apparatus (11) according to claim 1, wherein said probe controller (79) has an assistance mode for assisting laser radiation operation of said treatment probe (24),
in said assistance mode, said treatment probe permits said laser radiation operation while said lesion (17) exists, and prohibits said laser radiation operation when said lesion disappearance degree judgment section (69) judges that said lesion has disappeared.

13. The laser treatment apparatus (11) according to claim 12, further comprising:
a warning section for externally notifying said judgment result of said lesion disappearance degree judgment section (69).

14. The laser treatment apparatus (11) according to claim 1, wherein said laser beam has a wavelength of 5.75 µm.

15. The laser treatment apparatus (11) according to claim 1, wherein said laser beam has a wavelength between or equal to 1100 µm and 1800 µm.

16. The laser treatment apparatus (11) according to claim 1, wherein said laser beam radiates from a semiconductor laser or a free electron laser.

17. The laser treatment apparatus (11) according to claim 1, wherein said treatment probe (24) radiates said laser beam with a wavelength of at least one of 193 nm, 248 nm, 308 nm, and 351 nm.

18. The laser treatment apparatus (11) according to claim 1, wherein said treatment probe (24) radiates one of a plurality of laser beams with different wavelengths.
